# FASCICULE DE BREVET EUROPEEN

(11) **EP 1 673 121 B1**
(45) Date de publication et mention de la délivrance du brevet: **14.11.2018**
(21) Numéro de dépôt: 04787354.2
(22) Date de dépôt: 10.09.2004
(51) Int. Cl.: A61M 5/32

(54) **DISPOSITIF DE PROTECTION D`UN APPAREIL D`INJECTION**
VORRICHTUNG ZUM SCHUTZ EINER INJEKTIONSVORRICHTUNG
DEVICE FOR PROTECTING AN INJECTION DEVICE

(30) Priorité: 26.09.2003 FR 0311313
(43) Date de publication de la demande: 28.06.2006
(73) Titulaire: Becton Dickinson France, 38800 Le Pont-de-Claix (FR)
(72) Inventeur: BARRELLE, Laurent, 38250 Saint Nizier du Moucherotte (FR)
(74) Mandataire: Brédeville, Odile Marie
(86) Numéro de dépôt international: PCT/FR2004/002306
(87) Numéro de publication internationale: WO 2005/030301

(56) Documents cités:
- EP-A- 0 966 983
- EP-A1- 1 558 310
- FR-A- 2 830 765
- FR-A- 2 835 753
- US-A1- 2003 212 380

## Description

La présente invention concerne un dispositif de protection d'appareils d'injection d'un produit, notamment à usage médical, comme des seringues.

Dans la description ci-après, les termes "proximal " et "distal" sont considérés par rapport au sens d'injection du produit.

Les appareils d'injection de produit, comme les seringues, sont bien connus. Les seringues pré-remplies sont habituellement remplies par un médicament avant d'être distribuées à l'utilisateur final.

Toutefois, l'utilisateur final est constamment exposé aux risques de piqûres accidentelles susceptibles de se produire après l'injection.

Afin de réduire au maximum ces risques, il est connu de munir les seringues d'un dispositif de protection se présentant sous la forme d'un fourreau coulissant par rapport à la seringue et appelé à venir recouvrir l'aiguille après l'injection.

Certains de ces dispositifs doivent être mis en place manuellement par l'utilisateur final et sont de ce fait peu fiables. D'autres dispositifs de protection sont activés grâce à un ressort sur action de l'utilisateur final. Dans ce cas également, le déclenchement du dispositif de protection dépend d'une action de l'utilisateur final et est donc aléatoire.

Pour remédier à ces inconvénients, il existe des dispositifs de protection activés automatiquement par un ressort en fin d'injection. Un des problèmes rencontrés avec ces dispositifs est le risque de les activer prématurément ou par inadvertance, en particulier lors de leur fabrication et/ou de leur assemblage avec des seringues. On notera qu'il est connu du document EP0966983 un manchon de protection pour se ringue pré-remplie. Le document FR2835753 montre un dispositif de support de sécurité pour une seringue. Il est connu du document FR2830765 un dispositif de sécurité pour une seringue. Par ailleurs, les documents US2003212380 et EP1558310 divulguent des protections passives de sécurité pour des dispositifs d'injection.

Il existe donc un besoin d'un dispositif de protection d'une seringue qui puisse être activable automatiquement mais seulement en fin d'injection ou seulement lorsque l'utilisateur final le décide.

L'objectif de l'invention est donc de fournir un dispositif de protection d'un appareil d'injection, en particulier d'une seringue, activé automatiquement en fin d'injection mais ne pouvant être activé par inadvertance, assurant ainsi une parfaite sécurité contre les risques de piqûres accidentelles.

La présente invention porte sur un dispositif de protection selon la revendication 1.

Avantageusement, les moyens de coopération de la bague intermédiaire avec la tête de piston comprennent deux jambes diamétralement opposées s'étendant dans le sens proximal, légèrement décalées dans le sens radial par rapport au corps de la bague et reliées à l'extrémité proximale de la bague par des pont radiaux.

Avantageusement, les premiers moyens de retenue comprennent deux bourrelets longitudinaux diamétralement opposés et ménagés sur la surface interne de la paroi du corps du fourreau de support, chaque bourrelet comprenant à son extrémité proximale une rampe interne de retenue, et deux premières pattes s'étendant axialement dans le sens proximal à partir de l'extrémité proximale du fourreau de protection, chacune desdites premières pattes étant munie à son extrémité proximale d'une saillie dont la face distale est inclinée et apte à reposer sur la rampe interne de l'extrémité proximale d'un dit bourrelet. Ainsi, la coopération de la rampe interne de chaque bourrelet et de la surface distale de la saillie de la première patte en regard du bourrelet maintient le fourreau de protection dans sa première position dite d'injection dans sa configuration rentrée d'attente.

Avantageusement, les deuxièmes moyens de retenue comprennent une surface transversale de retenue, située à l'extrémité proximale de chaque bourrelet, en regard de la rampe interne dudit bourrelet, et deux deuxièmes pattes, s'étendant dans le sens proximal à partir de l'extrémité proximale du fourreau de protection, selon un axe légèrement incliné par rapport à l'axe longitudinal de l'appareil d'injection, chaque deuxième patte étant située en regard d'une dite première patte, chaque deuxième patte étant munie à son extrémité proximale d'un décrochement dont la face proximale est apte à reposer sur la surface transversale de retenue du bourrelet en regard. Ainsi, la coopération de la surface transversale de l'extrémité proximale du bourrelet et de la surface proximale du décrochement de la deuxième patte maintient le fourreau de protection dans sa deuxième position dite de fin d'injection dans sa configuration rentrée d'attente.

Avantageusement, les moyens de poussée sont sous la forme d'un ressort dont l'extrémité proximale prend appui sur l'extrémité distale de la bague intermédiaire et dont l'extrémité distale prend appui sur un rebord annulaire ménagé sur la surface interne du fourreau de protection au niveau de son extrémité proximale.

Les figures annexées illustrent, à titre d'exemple, un mode de réalisation préféré du dispositif selon l'invention.
La figure 1 en est une vue en perspective;
La figure 2 en est une vue en perspective avec la seringue assemblée,
La figure 3 est une vue en perspective éclatée montrant les éléments du dispositif selon l'invention,
Les figures 4 et 5 sont des vues de côté du dispositif selon l'invention dans sa configuration rentrée d'attente dans sa première position dite d'injection, respectivement avant et après assemblage de la seringue,
La figure 6 est une vue de côté du dispositif de l'invention en cours de désactivation des premiers moyens de retenue,
La figure 7 est une vue de côté du dispositif selon l'invention dans sa configuration rentrée d'attente dans sa deuxième position dite de fin d'injection,
La figure 8 est une vue de côté du dispositif selon l'invention en cours de désactivation des deuxièmes moyens de retenue,
La figure 9 est une vue de côté du dispositif selon l'invention dans sa configuration sortie de protection.

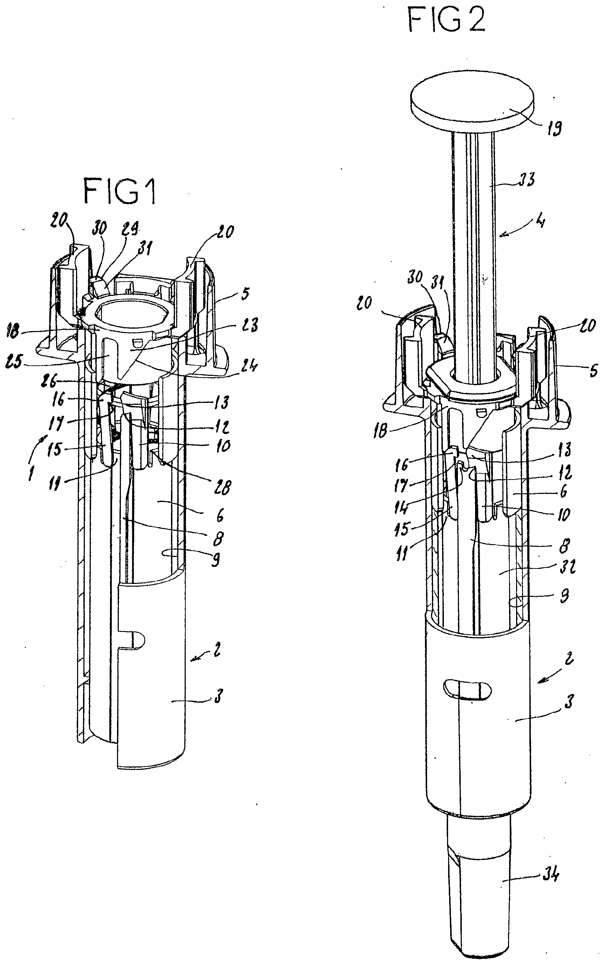

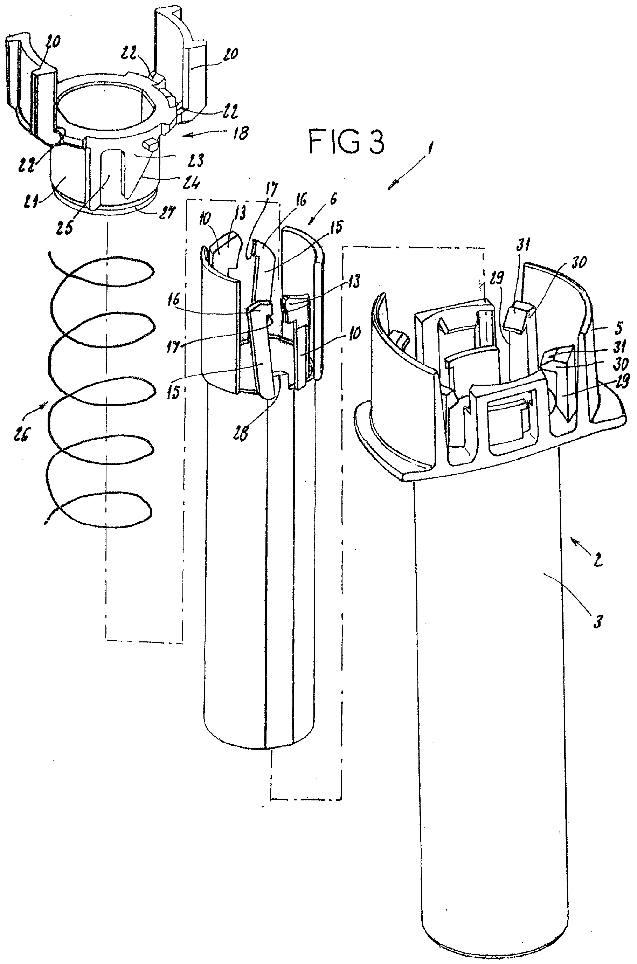

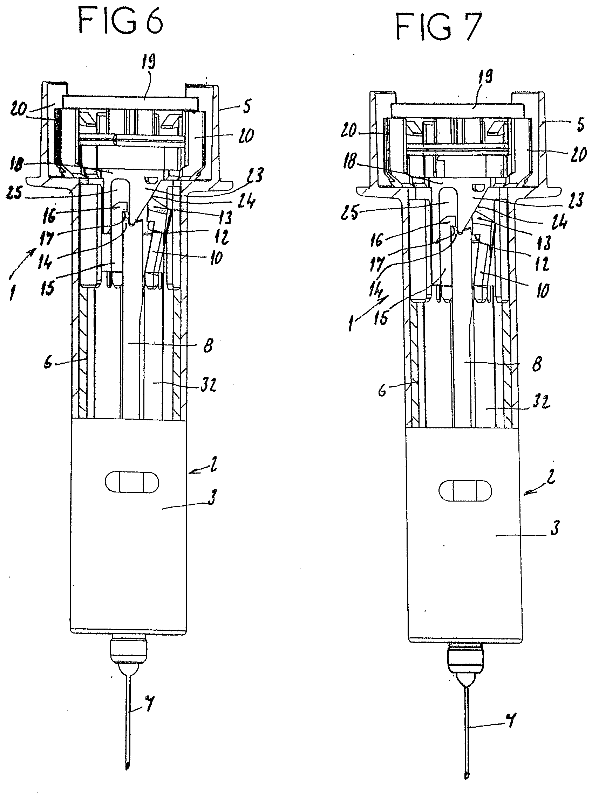

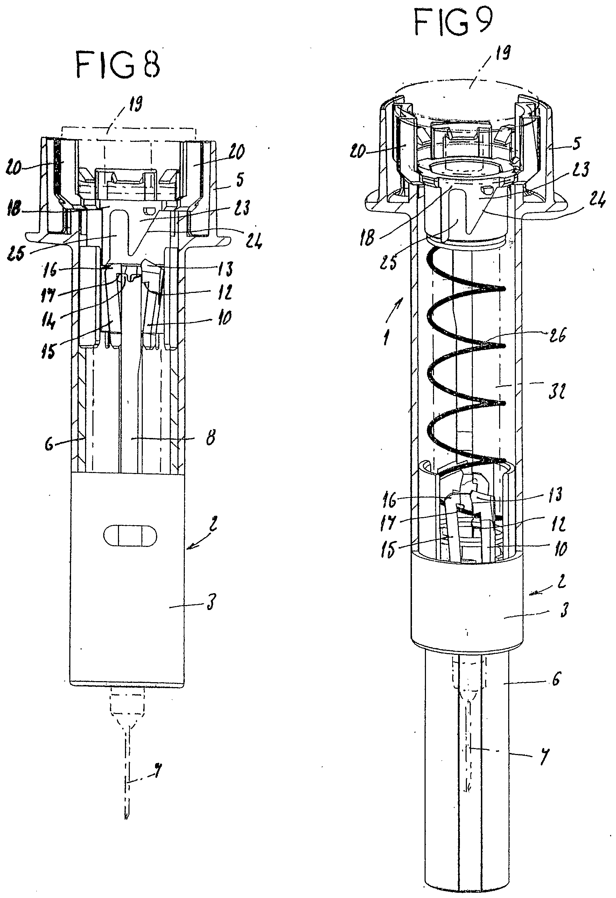

Sur les figures 1 à 3, est représenté un dispositif 1 de protection d'un appareil d'injection. Ce dispositif 1 comprend un fourreau de support 2 comprenant un corps 3 apte à recevoir un appareil d'injection 4, tel que la seringue montrée sur la figure 2 comprenant un réservoir 32, une tige 33 d'actionnement d'un piston, une tête 19 de piston et un capuchon 34 recouvrant une aiguille (voir figure 6). Le fourreau de support 2 comprend également une partie extrême proximale 5. Le dispositif 1 comprend également un fourreau de protection 6. Ce fourreau de protection 6 est susceptible de coulisser par rapport au fourreau de support 2 entre une configuration rentée d'attente dans laquelle l'aiguille 7 de l'appareil d'injection 4 est exposée, comme montré sur la figure 6, et une configuration sortie de protection dans laquelle le fourreau de protection 6 recouvre ladite aiguille 7 comme montré sur la figure 9.

Comme montré sur les figures 1 et 2, le dispositif 1 comprend des premiers moyens de retenue du fourreau de protection 6 dans sa configuration d'attente dans une première position, dite d'injection, sous la forme de deux bourrelets longitudinaux 8 ménagés sur la surface interne 9 de la paroi du corps 3 du fourreau de support 2 et de deux premières pattes 10 s'étendant axialement dans le sens proximal à partir de l'extrémité proximale 11 du fourreau de protection 6. De préférence, les bourrelets 8 sont diamétralement opposés. Chaque bourrelet 8 comprend à son extrémité proximale une rampe interne 12 de retenue et chaque première patte 10 est munie à son extrémité proximale d'une saillie 13 dont la face distale est inclinée et apte à reposer sur la rampe interne 12 de l'extrémité proximale du bourrelet 8 en regard. Comme il sera expliqué plus bas, ces premiers moyens de retenue du fourreau de protection 6 sont susceptibles d'être désactivés pour faire coulisser le fourreau de protection 6, dans sa configuration rentrée d'attente, entre une première position dite d'injection et une deuxième position, dite de fin d'injection.

Comme montré sur la figure 2, le dispositif 1 comprend également des deuxièmes moyens de retenue du fourreau de protection 6, dans sa configuration d'attente dans une deuxième position, dite de fin d'injection, sous la forme d'une surface transversale 14 de retenue, située à l'extrémité proximale de chaque bourrelet 8, en regard de la rampe interne 12 dudit bourrelet 8 et de deux deuxièmes pattes 15, s'étendant dans le sens proximal à partir de l'extrémité proximale 11 du fourreau de protection 6, selon un axe légèrement incliné par rapport à l'axe longitudinal de l'appareil d'injection 4, chaque deuxième patte 15 étant située en regard d'une dite première patte 10, chaque deuxième patte 15 étant munie à son extrémité proximale d'un décrochement 16 dont la face distale 17 est apte à reposer sur la surface transversale 14 de retenue du bourrelet 8 en regard. Comme il sera expliqué plus loin, ces deuxièmes moyens de retenue du fourreau de protection 6 sont susceptibles d'être désactivés pour autoriser la sortie du fourreau de protection 6 en fin d'injection.

Comme il ressort des figures 1 à 3, le dispositif 1 de protection comprend également une bague intermédiaire 18 située dans la partie extrême proximale 5 du fourreau de support 2. La partie extrême proximale 5 du fourreau de support 2 comprend des pattes 29, chaque patte 29 comprenant un décrochement radial 30 destiné à retenir la face proximale de la bague intermédiaire 18 dans le sens proximal, ledit décrochement radial 30 comprenant une rampe interne 31 dont la fonction sera expliquée plus loin. La bague intermédiaire 18 est susceptible de coulisser par rapport au fourreau de support 2 au sein de la partie extrême proximale 5 de ce fourreau de support 2. La bague intermédiaire 18 comprend des moyens de coopération avec la tête 19 de piston de l'appareil d'injection 4. Dans l'exemple représenté, ces moyens de coopération se présentent sous la forme de deux jambes 20 diamétralement opposées s'étendant dans le sens proximal, légèrement décalées dans le sens radial par rapport au corps 21 de la bague 18 et reliées à l'extrémité proximale de la bague 18 par des ponts radiaux 22.

La bague intermédiaire 18 comprend également des moyens de désactivation des premiers et deuxièmes moyens de retenue, sous la forme dans l'exemple représenté, d'une surface 23 saillant radialement du corps 21 de la bague 18, cette surface 23 étant apte à coopérer avec les dites premières pattes 10 et lesdites deuxièmes pattes 15 pour les défléchir de façon circonférentielle. Dans l'exemple représenté, cette surface 23 présente une rampe externe 24 en regard de chaque première patte 10 et un évidement longitudinal 25 en regard de chaque deuxième patte 15.

Le dispositif 1 de protection comprend également au moins un moyen de poussée, sous la forme, dans l'exemple représenté, d'un ressort 26 dont l'extrémité proximale prend appui sur l'extrémité distale 27 de la bague intermédiaire 18 et dont l'extrémité distale prend appui sur un rebord annulaire 28 ménagé sur la surface interne du fourreau de protection 6 au niveau de son extrémité proximale 11.

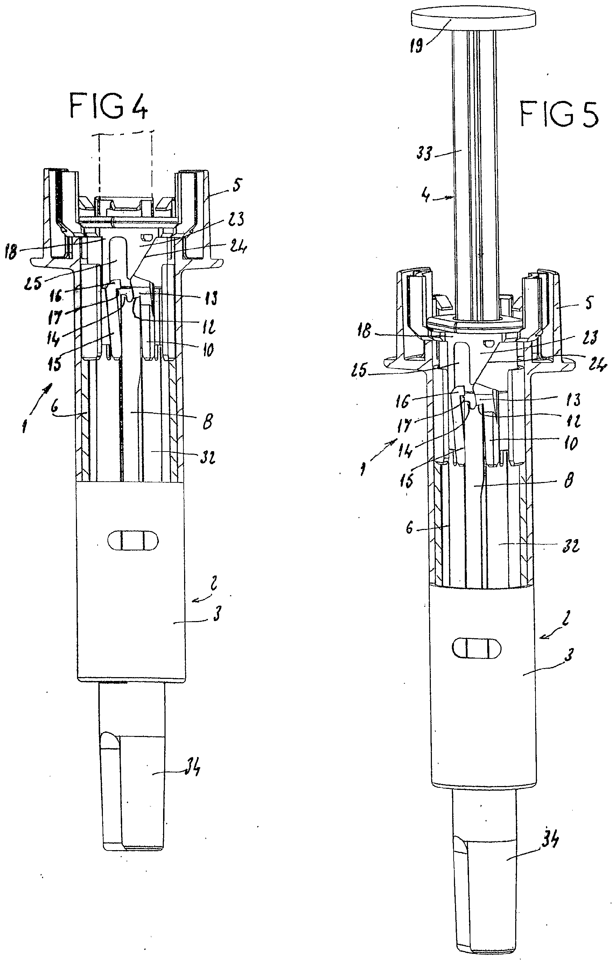

En pratique, le dispositif 1 de protection selon l'invention est dans la position de stockage telle que représentée à la figure 4. Le fourreau de protection 6 a été inséré au sein du fourreau de support 2 jusqu'à ce que les faces distales respectives des saillies 13 des premières pattes 10 parviennent au contact des rampes internes 12 de retenue respectives des bourrelets 8. Du fait de leur légère inclinaison par rapport à l'axe longitudinal du dispositif 1, les faces distales 17 des décrochements 16 des deuxièmes pattes 15 ne sont pas en contact avec les surfaces transversales 14 des bourrelets 8. Puis le ressort 26 a ensuite été inséré, son extrémité distale prenant appui sur le rebord annulaire 28 du fourreau de protection 6. La bague intermédiaire 18 a ensuite été insérée par pression sur les rampes internes 31 des décrochements radiaux 30 des pattes 29 qui se sont défléchies au passage de ladite bague 18. En position de stockage, la bague intermédiaire 18 est donc encliquetée au sein de la partie extrême proximale 5 du fourreau de support 2 au moyen des pattes 29 et est retenue dans le sens proximal par les décrochements radiaux 30 de ces pattes 29. L'extrémité proximale du ressort 26 prend appui sur l'extrémité distale 27 de la bague intermédiaire 18. Le système est ainsi parfaitement verrouillé, sans risque de déclenchement de l'activation du fourreau de protection. Dans cette position, l'appareil d'injection 4, sous la forme d'une seringue dans l'exemple représenté, est assemblé comme montré sur la figure 5 et l'injection du produit contenu dans la seringue peut avoir lieu.

En fin d'injection, comme montré sur la figure 6, la tête 19 de piston de l'appareil d'injection 4 vient au contact des moyens de coopération, c'est-à-dire des jambes 20 sur l'exemple représenté, de la bague intermédiaire 18. En continuant à exercer une pression et à pousser sur la tête 19 de piston, la bague intermédiaire 18 est déplacée dans le sens distal et les rampes externes 24 de la surface 23 saillant radialement du corps 21 de la bague 18 défléchissent les premières pattes 10 de façon circonférentielle. Simultanément, les décrochements 16 des deuxièmes pattes 15 sont guidés dans les évidements longitudinaux 25 de la surface 23 saillant radialement du corps 21 de la bague intermédiaire 18 et les deuxièmes pattes 15 sont ainsi défléchies de façon circonférentielle pour se retrouver parallèles aux bourrelets 8.

Ainsi, les premiers moyens de retenue du fourreau de protection 6 dans sa configuration rentrée d'attente dans la première position dite d'injection sont désactivés et, sous la pression du ressort 26, le fourreau de protection 6 est déplacé dans le sens distal, sur une faible distance, jusqu'à ce que les faces distales 17 des décrochements 16 des deuxièmes pattes 15, guidées par les évidements longitudinaux 25, parviennent au contact des surfaces transversales 14 de retenue des bourrelets 8, comme montré sur la figure 7. Le fourreau de protection 6 est alors dans sa configuration rentrée d'attente dans la deuxième position dite de fin d'injection. Dans cette position, le dispositif 1 de protection est bloqué. Le piston est en bout de course et il n'est pas possible de déclencher l'activation du fourreau de protection 6 en continuant à pousser sur la tête 19 de piston.

Pour déclencher l'activation du fourreau de protection, l'utilisateur doit relâcher faiblement sa pression sur la tête 19 de piston. Ainsi, à ce stade, l'utilisateur final peut décider d'activer le fourreau de protection 6 alors que l'aiguille 7 est encore dans le patient ou peut au contraire décider de retirer l'aiguille 7 du patient puis d'activer le fourreau de protection 6.

Lorsque l'utilisateur relâche faiblement la pression sur la tête 19 de piston, la bague intermédiaire 18 est déplacée dans le sens proximal sous l'effet de la poussée du ressort 26. Ce faisant, la surface 23 saillant radialement du corps 21 de la bague 18 libère les deuxièmes pattes 15 comme montré sur la figure 8. Ces deuxièmes pattes 15 reprennent leur position initiale légèrement inclinée par rapport à l'axe longitudinal du dispositif 1 et les faces distales 17 des décrochements 16 n'appuient plus sur les surfaces transversales 14 de retenue des bourrelets 8.

Sous l'effet de la poussée du ressort 26, le fourreau de protection 6 est alors déplacé dans le sens distal et vient recouvrir l'aiguille 7 (en traits mixtes) comme montré sur la figure 9.

Il apparaît de ce qui précède que l'invention apporte des améliorations déterminantes aux dispositifs homologues de la technique antérieure, en permettant d'activer le fourreau de protection seulement en fin d'injection et au moment où l'utilisateur final le décide.

Il va de soi que l'invention n'est pas limitée à la forme de réalisation décrite ci-dessus à titre d'exemple mais qu'elle en embrasse au contraire toutes les variantes de réalisation entrant dans le champ de protection défini par les revendications ci-annexées.

## Revendications

1. Dispositif (1) de protection d'un appareil d'injection (4) d'un produit, en particulier une seringue, ledit appareil comprenant un réservoir (32) muni d'une aiguille (7) à son extrémité distale et un piston relié à une tige d'actionnement surmontée d'une tête (19) de piston, ledit dispositif (1) comprenant :
- un fourreau de support (2) comprenant un corps (3) apte à recevoir l'appareil d'injection (4) et une partie extrême proximale (5),
- un fourreau de protection (6) susceptible de coulisser par rapport au fourreau de support (2) entre une configuration rentrée d'attente dans laquelle l'aiguille (7) est exposée et une configuration sortie de protection dans laquelle il recouvre l'aiguille (7),
- des premiers moyens de retenue (8, 10, 12, 13) du fourreau de protection (6) dans sa configuration d'attente dans une première position, dite position d'injection,
- des deuxièmes moyens de retenue (8, 14-17) du fourreau de protection (6) dans sa configuration d'attente dans une deuxième position, dite position de fin d'injection, sensiblement décalée dans le sens distal, par rapport au fourreau de support (2),
- une bague intermédiaire (18) située dans la partie extrême proximale (5) du fourreau de support (2), susceptible de coulisser par rapport à ce fourreau de support (2) au sein de ladite partie extrême proximale (5), ladite bague (18) comprenant des moyens de coopération (20) avec la tête (19) de piston de l'appareil d'injection (4), et des moyens de désactivation (23-25) desdits premiers et deuxièmes moyens de retenue (8, 10, 12-17),
- lesdits premiers moyens de retenue (8, 10, 12, 13) étant susceptibles d'être désactivés par lesdits moyens de désactivation (23-25) de ladite bague intermédiaire (18), par pression de la tête (19) de piston dans le sens distal sur lesdits moyens de coopération (20) de ladite bague intermédiaire (18), pour faire coulisser le fourreau de protection (6), dans sa configuration rentrée d'attente, entre ladite première position d'injection et ladite deuxième position de fin d'injection,
- et lesdits deuxièmes moyens de retenue (8, 14-17) étant susceptibles d'être désactivés par les moyens de désactivation (20) de ladite bague intermédiaire (18), par relâchement de la pression de la tête (19) de piston sur lesdits moyens de coopération (20) de ladite bague intermédiaire (18), pour autoriser la sortie du fourreau de protection (6) sous l'action de moyens de poussée (26),
- **caractérisé en ce que** les moyens de désactivation (23-25) comprennent une surface (23) saillant radialement du corps (21) de la bague (18), cette surface (23) étant apte à coopérer avec des premières pattes (10) des premiers moyens de retenue et avec des deuxièmes pattes (15) des deuxièmes moyens de retenue pour défléchir lesdites premières pattes (10) et lesdites deuxièmes pattes (15) de façon circonférentielle.

2. Dispositif (1) selon la revendication 1, **caractérisé en ce que** les moyens de coopération (20) de la bague intermédiaire (18) avec la tête (19) de piston comprennent deux jambes (20) diamétralement opposées s'étendant dans le sens proximal, légèrement décalées dans le sens radial par rapport au corps (21) de la bague (18) et reliées à l'extrémité proximale de la bague (18) par des pont radiaux (22).

3. Dispositif (1) selon la revendication 1 ou 2, **caractérisé en ce que** les premiers moyens de retenue (8, 10, 12, 13) comprennent deux bourrelets (8) longitudinaux diamétralement opposés et ménagés sur la surface interne (9) de la paroi du corps (3) du fourreau de support (2), chaque bourrelet (8) comprenant à son extrémité proximale une rampe interne de retenue (12), et deux premières pattes (10) s'étendant axialement dans le sens proximal à partir de l'extrémité proximale (11) du fourreau de protection (6), chacune desdites premières pattes (10) étant munie à son extrémité proximale d'une saillie (13) dont la face distale est inclinée et apte à reposer sur la rampe interne (12) de l'extrémité proximale d'un dit bourrelet (8).

4. Dispositif (1) selon la revendication 3, **caractérisé en ce que** les deuxièmes moyens de retenue (8, 14-17) comprennent une surface transversale (14) de retenue, située à l'extrémité proximale de chaque bourrelet (8), en regard de la rampe interne (2) dudit bourrelet (8), et deux deuxièmes pattes (15), s'étendant dans le sens proximal à partir de l'extrémité proximale du fourreau de protection (6), selon un axe légèrement incliné par rapport à l'axe longitudinal de l'appareil d'injection (4), chaque deuxième patte (15) étant située en regard d'une dite première patte (10), chaque deuxième patte (15) étant munie à son extrémité proximale d'un décrochement (16) dont la face distale (17) est apte à reposer sur la surface transversale (14) de retenue du bourrelet (8) en regard.

5. Dispositif (1) selon l'une quelconque des revendications précédentes, **caractérisé en ce que** les moyens de poussée (26) sont sous la forme d'un ressort (26) dont l'extrémité proximale prend appui sur l'extrémité distale (27) de la bague intermédiaire (18) et dont l'extrémité distale prend appui sur un rebord annulaire (28) ménagé sur la surface interne du fourreau de protection (6) au niveau de son extrémité proximale.

## Patentansprüche

1. Vorrichtung (1) zum Schützen eines Geräts zum Injizieren (4) eines Produkts, insbesondere einer Spritze, wobei das Gerät ein Reservoir (32) umfasst, das an seinem distalen Ende mit einer Nadel (7) ausgestattet ist, und einen Kolben, der mit einer Betätigungsstange verbunden ist, welche von einem Kolbenkopf (19) überragt wird, wobei die Vorrichtung (1) umfasst:
- ein Haltefutteral (2), umfassend einen Körper (3), der in der Lage ist, das Injektionsgerät (4) aufzunehmen, und einen proximalen Endteil (5),
- ein Schutzfutteral (6), das in Bezug auf das Haltefutteral (2) zwischen einer eingefahrenen Wartekonfiguration, in der die Nadel (7) freiliegt, und einer ausgefahrenen Schutzkonfiguration gleiten kann, in der es die Nadel (7) bedeckt,
- erste Mittel zum Zurückhalten (8, 10, 12, 13) des Schutzfutterals (6) in seiner Wartekonfiguration in einer Injektionsstellung genannten ersten Stellung,
- zweite Mittel zum Zurückhalten (8, 14-17) des Schutzfutterals (6) in seiner Wartekonfiguration in einer Injektionsendstellung genannten zweiten Stellung, die in Bezug auf das Haltefutteral (2) im Wesentlichen in der distalen Richtung verschoben ist,
- einen im proximalen Endteil (5) des Haltefutterals (2) liegenden Zwischenring (18), der in Bezug auf dieses Haltefutteral (2) innerhalb des proximalen Endteils (5) gleiten kann, wobei der Ring (18) Mittel zum Zusammenwirken (20) mit dem Kolbenkopf (19) des Injektionsgeräts (4), und Mittel zum Deaktivieren (23-25) der ersten und zweiten Rückhaltemittel (8, 10, 12-17) umfasst,
- wobei die ersten Rückhaltemittel (8, 10, 12, 13) von den Deaktivierungsmitteln (23-25) des Zwischenrings (18) deaktiviert werden können durch Druck des Kolbenkopfes (19) in der distalen Richtung auf die Zusammenwirkmittel (20) des Zwischenrings (18), um das Schutzfutteral (6) in seiner eingefahrener Wartekonfiguration zwischen der ersten Injektionsstellung und der zweiten Injektionsendstellung gleiten zu lassen,
- und die zweiten Rückhaltemittel (8, 14-17) von den Deaktivierungsmitteln (23-25) des Zwischenrings (18) deaktiviert werden können durch Lösendes Drucks des Kolbenkopfes (19) auf die Zusammenwirkmittel (20) des Zwischenrings (18), um das Ausfahren des Schutzfutterals (6) unter der Wirkung von Schubmitteln (26) zu gestatten,
- **dadurch gekennzeichnet, dass** die Deaktivierungsmittel (23-25) eine Fläche (23) umfassen, die radial vom Körper (21) des Rings (18) vorspringt, wobei diese Fläche (23) in der Lage ist, mit ersten Klauen (10) der ersten Rückhaltemittel, und mit zweiten Klauen (15) der zweiten Rückhaltemittel zusammenzuwirken, um die ersten Klauen (10) und die zweiten Klauen (15) umfangsmäßig abzulenken.

2. Vorrichtung (1) nach Anspruch 1, **dadurch gekennzeichnet, dass** die Mittel zum Zusammenwirken (20) des Zwischenrings (18) mit dem Kolbenkopf (19) zwei sich diametral gegenüberliegende Schenkel (20) umfassen, die sich in der proximalen Richtung erstrecken, in Bezug auf den Körper (21) des Rings (18) leicht in der radialen Richtung verschoben und über radiale Stege (22) mit dem proximalen Ende des Rings (18) verbunden sind.

3. Vorrichtung (1) nach Anspruch 1 oder 2, **dadurch gekennzeichnet, dass** die ersten Rückhaltemittel (8, 10, 12, 13) zwei Längssicken (8) umfassen, die sich diametral gegenüberliegen und an der Innenfläche (9) der Wand des Körpers (3) des Haltefutterals (2) ausgestaltet sind, wobei jede Sicke (8) an ihrem proximalen Ende eine innere Rückhalterampe (12) umfasst, und zwei erste Klauen (10), die sich axial in der proximalen Richtung ab dem proximalen Ende (11) des Schutzfutterals (6) erstrecken, wobei jede der ersten Klauen (10) an ihrem proximalen Ende mit einem Vorsprung (13) ausgestatten ist, dessen distale Seite geneigt und in der Lage ist, an der inneren Rampe (12) des proximalen Endes einer sogenannten Sicke (8) zu ruhen.

4. Vorrichtung (1) nach Anspruch 3, **dadurch gekennzeichnet, dass** die zweiten Rückhaltemittel (8, 14-17) eine Quer-Rückhaltefläche (14) umfassen, die am proximalen Ende jeder Sicke (8) der inneren Rampe (2) der Sicke (8) zugewandt liegt, und zwei zweite Klauen (15), die sich in der proximalen Richtung ab dem proximalen Ende des Schutzfutterals (6) entlang einer Achse erstrecken, die in Bezug auf die Längsachse des Injektionsgeräts (4) leicht geneigt ist, wobei jede zweite Klaue (15) einer sogenannten ersten Klaue (10) zugewandt liegt, wobei jede zweite Klaue (15) an ihrem proximalen Ende mit einer Kröpfung (16) ausgestattet ist, deren distale Seite (17) in der Lage ist, an der Quer-Rückhaltefläche (14) der zugewandten Sicke (8) zu ruhen.

5. Vorrichtung (1) nach einem der vorstehenden Ansprüche, **dadurch gekennzeichnet, dass** die Schubmittel (26) in der Form einer Feder (26) sind, deren proximales Ende am distalen Ende (27) des Zwischenrings (18) in Anlage geht, und deren distales Ende an einem ringförmigen Rand (28) in Anlage geht, der an der inneren Fläche des Schutzfutterals (6) im Bereich seines proximalen Endes ausgestaltet ist.

## Claims

1. A device (1) for protecting a product injection apparatus (4), particularly a syringe, said apparatus comprising a reservoir (32) provided with a needle (7) at its distal end and a piston connected to an actuator rod surmounted by a piston head (19), said device (1) comprising:
- a support sheath (2) comprising a body (3) able to receive the injection apparatus (4) and a proximal end portion (5),
- a protective sheath (6) likely to slide with respect to the support sheath (2) between a retracted standby configuration in which the needle (7) is exposed and an extended protection configuration in which it covers the needle (7)
- first means (8, 10, 12, 13) for retaining the protective sheath (6) in its standby configuration in a first position, called injection position,
- second means (8, 14-17) for retaining the protective sheath (6) in its standby configuration in a second position, called end-of-injection position, substantially offset in the distal direction, with respect to the support sheath (2),
- an intermediate ring (18) located in the proximal end portion (5) of the support sheath (2), likely to slide with respect to this support sheath (2) within said proximal end portion (5), said ring (18) comprising means (20) for cooperation with the piston head (19) of the injection apparatus (4), and means (23-25) for deactivating said first and second retaining means (8, 10, 12-17),
- said first retaining means (8, 10, 12, 13) being likely to be deactivated by said means (23-25) for deactivating said intermediate ring (18), by pressing the piston head (19) in the distal direction on said cooperation means (20) of said intermediate ring (18), for sliding the protective sheath (6), in its retracted standby configuration, between said first injection position and said second end-of-injection position,
- and said second retaining means (8, 14-17) being likely to be deactivated by the means (23-25) for deactivating said intermediate ring (18), by releasing the pressure of the piston head (19) on said cooperation means (20) of said intermediate ring (18), in order to allow the exit of the protective sheath (6) under the action of pushing means (26),
- **characterized in that** the deactivation means (23-25) comprise a surface (23) radially protruding from the body (21) of the ring (18), this surface (23) being able to cooperate with first tabs (10) of the first retaining means and with second tabs (15) of the second retaining means in order to deflect said first tabs (10) and said second tabs (15) circumferentially.

2. The device (1) according to claim 1, **characterized in that** the means (20) of the intermediate ring (18) for cooperation with the piston head (19) comprise two diametrically opposed legs (20) extending in the proximal direction, slightly offset in the radial direction with respect to the body (21) of the ring (18) and connected to the proximal end of the ring (18) by radial bridges (22).

3. The device (1) according to claim 1 or 2, **characterized in that** the first retaining means (8, 10, 12, 13) comprise two diametrically opposed longitudinal beads (8) formed on the inner surface (9) of the wall of the body (3) of the support sheath (2), each bead (8) comprising, at its proximal end, an inner retaining ramp (12), and two first tabs (10) extending axially in the proximal direction from the proximal end (11) of the protective sheath (6), each of said first tabs (10) being provided at its proximal end with a protrusion (13) whose distal face is inclined and able to rest on the inner ramp (12) of the proximal end of one said bead (8).

4. The device (1) according to claim 3, **characterized in that** the second retaining means (8, 14-17) comprise a transverse retaining surface (14) located at the proximal end of each bead (8), facing the inner ramp (2) of said bead (8), and two second tabs (15), extending proximally from the proximal end of the protective sheath (6), along an axis slightly inclined relative to the longitudinal axis of the injection apparatus (4), each second tab (15) being located facing one said first tab (10), each second tab (15) being provided, at its proximal end, with a recess (16) whose distal face (17) is able to rest on the transverse retaining surface (14) of the opposite bead (8).

5. The device (1) according to any one of the preceding claims, **characterized in that** the pushing means (26) are in the form of a spring (26) whose proximal end bears on the distal end (27) of the intermediate ring (18) and whose distal end bears on an annular rim (28) formed on the inner surface of the protective sheath (6) at its proximal end.
